(19) Europäisches Patentamt
European Patent Office
Office européen des brevets

(11) EP 2 493 575 B1

(12) EUROPEAN PATENT SPECIFICATION

(45) Date of publication and mention of the grant of the patent:
**17.02.2016 Bulletin 2016/07**

(21) Application number: **10774381.7**

(22) Date of filing: **26.10.2010**

(51) Int Cl.:
***A61K 8/29*** *(2006.01)* ***A61K 8/37*** *(2006.01)*
***A61Q 1/10*** *(2006.01)* ***A61K 8/92*** *(2006.01)*

(86) International application number:
**PCT/US2010/054085**

(87) International publication number:
**WO 2011/056565 (12.05.2011 Gazette 2011/19)**

# (54) METHOD OF COATING EYELASHES AND EYELIDS WITH A LONG-WEARING MASCARA COMPOSITION

VERFAHREN ZUR BESICHTIGUNG VON WIPPERN MIT EINER LANGHALTENDEN MASCARA ZUSAMMENSETZUNG

PROCÉDÉ D'ENROBAGE DES CILS ET PAUPIÈRES PAR UNE COMPOSITION DE MASCARA DE LONGUE DURÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2009 US 255451 P**

(43) Date of publication of application:
**05.09.2012 Bulletin 2012/36**

(73) Proprietor: **The Procter & Gamble Company**
**Cincinnati, OH 45202 (US)**

(72) Inventors:
- **DEMPSEY, James, Herman**
**Forest Hill**
**Maryland 21050 (US)**
- **RABE, Thomas, Elliot**
**Baltimore**
**Maryland 21218 (US)**

(74) Representative: **Engisch, Gautier**
**NV Procter & Gamble Services Company SA**
**IP Patent Department**
**Temselaan 100**
**1853 Strombeek-Bever (BE)**

(56) References cited:
**WO-A1-2007/040517**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 493 575 B1

## Description

### FIELD OF THE INVENTION

This invention relates to long-wearing mascara compositions and methods of coating eyelashes, eyebrows, and/or eyelids with the same. The mascara composition may be formulated for use as an eyeliner or brow product. The mascara can last on the eyelashes and/or eyelids in excess of 24 hours and in certain embodiments in excess of 48 hours. The methods of the present invention can include applying mascara to the eyelashes as a single layer application or as a first layer with a top coat. A solvent-based removal composition is also disclosed because the mascara compositions of the present invention have very low solubility in soap and water.

### BACKGROUND OF THE INVENTION

Mascara compositions have been used for many years to increase the thickness, length and overall appearance of human eyelashes. There are generally two types of mascara: regular and water proof. Regular mascara is generally water soluble, while the water proof variety can be removed with soap and water. Both are typically applied only once per day. As the day progresses, the mascara gradually wears off. Current mascaras wear off relatively quickly and reapplication during the day is necessary but inconvenient. Moreover, current mascara is easily worn off by physical abrasion. If the small amount of mascara remaining on the eyelashes at night is not removed, it will gradually rub off during the night onto the sleeping surface leaving an undesirable mess. Women prefer to have the look that mascara provides at all times during the day. But the application process can be time consuming and inconvenient. Thus, women often sacrifice the look they desire because they simply do not have the time, or are not able to continually reapply mascara.

"Long Wear" mascaras are also available. But "long" is a relative term, and existing long wear mascaras are designed to eliminate reapplication during the day, and are generally not suitable for multi-day wear. One of the problems with long wear mascara is that making the composition sufficiently sticky to stay on the lashes, it is also sufficiently sticky to cause excessive clumping of the lashes. Some clumping is inevitable, but if too many individual lashes stick together and appear as large clumps, the consumer does not get the desired look.

Two layer mascaras are known, but the top coat is typically a visual agent. That is, the second coat compliments the first, or brings a second color. But these are typically not intended for wear beyond the normal 12-16 hour day. And these two layer mascaras are typically removed at night with soap and water.

There exists a need for methods of applying a mascara composition, either one or two layers, that can last overnight on a person’s eyes without degradation and without wearing off on sheets, pillow cases and other bed clothes. There is a need for a method of applying a mascara composition that lasts more than 24 hours and achieves the desired look of current mascaras and the consumer desired feel. Further there is a need for a solvent-based mascara remover that can remove mascara compositions that are not generally soluble in soap and water. The methods and compositions of the present invention meet these and other needs.

### SUMMARY OF THE INVENTION

In one aspect of the present invention there is provided a method of coating eyelids and eyelashes comprising the step of applying to eyelashes, eyelids, or both, a mascara composition. The mascara composition comprising: from 15 to 50 %, by weight, of a component selected from the group consisting of tall oil glyceride, pentaerythrityl rosinate, glyceryl rosinate, and the hydrogenated versions and mixtures thereof and from 0.5 to 50%, by weight, of a colorant. The mascara composition is substantially free of wax.

Further, there is provided a method of coating eyelids and eyelashes comprising the steps of applying to eyelashes, eyelids, or both, a mascara composition comprising a two layer cosmetic composition. The cosmetic composition comprises a first and second layer. The first layer comprising from about 15% to about 35%, by weight, of a film former selected from the group consisting of tall oil glyceride, pentaerythrityl rosinate, glyceryl rosinate, and the hydrogenated versions and mixtures thereof; and from about 0.1% to about 14%, by weight, of a colorant. The second layer comprises a clear polymer selected from the group consisting of organosiloxane resins and mixtures thereof.

### DETAILED DESCRIPTION OF THE INVENTION

All percentages are by weight of the personal-care composition, unless otherwise specified. All ratios are weight ratios, unless specifically stated otherwise. All numeric ranges are inclusive of narrower ranges; delineated upper and lower range limits are interchangeable to create further ranges not explicitly delineated. The number of significant digits conveys neither limitation on the indicated amounts nor on the accuracy of the measurements. All measurements are understood to be made at 25 °C and at ambient conditions, where "ambient conditions" means conditions under about

one atmosphere of pressure and at 50% relative humidity.

"Mascara" and "mascara composition," as used herein, mean a compound that is applied to eyelashes, eyelids, and/or eyebrows. Mascara compositions of the present invention may be formulated for topical application on mammalian keratinous tissue for use in skin-care, cosmetic, and hair-care products; non-limiting uses of which include antiperspirants, deodorants, lotions (e.g. hand lotion and body lotion), skin-care products (e.g., face and neck lotions, serums, sprays), sunless tanners, cosmetics (e.g., foundation, mascara, concealer, blush, lipstick, lip gloss, eyeliner, eye shadow, eyebrow pencil), hair dyes, after-shaves, razor moisturizing/lubricating strips, razor shave-gel bars, feminine-care products, oral-care products, and baby-care products. The methods of using any of the aforementioned compositions are also included within the meaning of mascara composition. "Keratinous tissue," as used herein, refers to keratin-containing layers disposed as the outermost protective covering of mammals which includes, but is not limited to, skin, hair, and nails.

***Mascara Composition***

The present invention relates to a method of coating eyelids and eyelashes comprising the step of applying to eyelashes, eyelids, or both, a mascara composition. The mascara composition remains intact on the eyelashes and/or eyelids of the wearer for greater than 24 hours, preferably greater than 36 hours, and even more preferably greater than about 48 hours. Solvent-based mascara-remover compositions are disclosed below because the mascara composition is generally not soluble in soap and water. The mascara composition comprises at least 15%, preferably at least 17%, and even more preferably at least 20% to 35%, by weight, of a film former selected from the group consisting of tall oil glyceride, pentaerythrityl rosinate, glyceryl rosinate, and the hydrogenated versions and mixtures thereof. The ratio of film former to volatile solvent is controlled such that the dried film consists of from 30% to 70% film former by weight. Other ingredients suitable for use in the compositions of the present invention are described and exemplified below.

A second essential component of the mascara composition is a colorant, which is described and exemplified below. Common colorants suitable for use in the mascaras compositions of the present invention include dyes and pigments. The colorant comprises at least 0.5%, and preferably at least 1.0% to 14%, by weight, of the mascara composition. The mascara compositions can be a single coat application, a base coast of mascara and a top coat. Both embodiments, single coat and two coat, can be used with the solvent-based remover composition described below.

Similar to many "waterproof" mascara formulas, the inventive mascara is an anhydrous dispersion (not emulsion) comprised of colorant particles dispersed in a matrix of film former(s) and volatile solvent(s). Key compositional elements and features are outlined below.

***Film Formers***

An essential component of the invention is a film former from a class of rosinates or rosin esters. The mascara composition comprises film formers chosen from the group consisting of tall oil glycerides, pentaerythrityl rosinate, glyceryl rosinate, and mixtures thereof. These materials are derived from rosin. Rosin is a solid form of resin obtained from conifers, produced by heating liquid resin to vaporize the volatile liquid terpene components. It primarily consists of different resin acids, especially abietic acid. Rosin is typically obtained commercially either by distillation of volatile turpentine from oleoresin exuded from the wound of living pine trees to obtain gum rosin or the separation of tall oil, a byproduct of the wood pulp industry, to obtain tall oil rosin.

The carboxylic acid group of a rosin acid can be converted to an ester through a reaction with various alcohols. Esterification of rosin modifies the softening point, adhesiveness, cohesiveness, and melted viscosity of the material. The alcohols typically used to make rosin esters are methanol, tri-ethylene-glycol, glycerol, and pentaerythritol. Tall oil rosin is esterified with glycerol to form tall oil glycerides, a mixture of resin acids, rosin acids, and esters of glycerol. Tall oil glycerides are available from, for example, Arizona Chemical Co. Glyceryl Rosinate is the ester of rosin acids with glycerol. Pentaerythrityl rosinate, sometimes referred to as pentaerythritol rosinate, is the ester of rosin acids with pentaerythritol. It is used as a skin conditioning agent-emollient and viscosity increasing agent-nonaqueous in a few cosmetic formulations. Pentaterythrityl Rosinate is commercially available, for example, from Eastman.

Rosin's conjugated double bond makes it susceptible to oxidation, isomerization and other reactions. A common method to improve stability is to hydrogenate the rosin molecules. This is done by the addition of hydrogen to double bonds in the resin acid, typically catalyzed by nickel compounds or noble metals to form saturated ring structures. Hydrogenation greatly increases the molecule's resistance to oxidation and improves its color. The hydrogenation process can be controlled so that the rosin is either partially or fully hydrogenated. Hydrogenated rosins have specific advantages over non-hydrogenated rosin resins including lighter color, improved stability, and reduced skin sensitization. The hydrogenated versions of pentaerythrityl rosinate and glyceryl rosinate, pentaerythrityl hydrogenated rosinate (PHR) and glyceryl hydrogenated rosinate (GHR) may be used in the invention.

Film formers such as tall oil glycerides, pentaerythrityl rosinate, pentaerythrityl hydrogenated rosinate, glyceryl rosinate, and glyceryl hydrogenated rosinate are used in the present invention in higher concentration than previously

used in mascara. The combination of film formers is from 15% to 50%, preferably from 20% to 30%, more preferably from 25% to 30%. The ratio of film former (e.g., PHR, GHR, tall oil glycerides, and combinations thereof) to volatile carrier is controlled such that the dried film consists of from 30% to 70% film former by weight, preferably from 40% to 60 %.

***Carrier Solvents***

The mascara composition may comprise a carrier solvent to achieve delivery of the film formers to the eyelash or eyelid. In a preferred embodiment, the mascara composition comprises a volatile carrier which quickly volatilizes from the surface of the eyelashes or eyelid, leaving the desired components behind.

The volatile carrier comprises from 10% to 85%, preferably from 15% to 80%, and most preferably from 20% to 70% of the composition. The volatile carrier of the present invention is selected from the group consisting of volatile hydrocarbons, volatile silicones and mixtures thereof.

Hydrocarbon oils useful in the present invention include those having boiling points in the range of 60-260°C, more preferably hydrocarbon oils having from about C8 to about C20 chain lengths, most preferably C8to C20 isoparaffins. Of these isopariffins most preferred are selected from the group consisting of isododecane, isohexadecane, isoeicosane, 2,2,4-trimethylpentane, 2,3-dimethylhexane and mixtures thereof. Most preferred is isododecane, available as for example Permethyl 99A from Presperse corresponding to the formula:

CH3(CH2)10CH

Preferred volatile silicone fluids include cyclomethicones having 3, 4 and 5 membered ring structures corresponding to the formula:

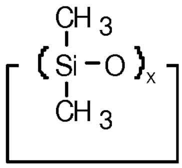

where X is from about 3 to about 6. Said volatile silicones include 244 Fluid, 344 Fluid and 245 Fluid, and 345 Fluid all from Dow Corning Corporation.

***Colorants***

Colorants suitable for use in the present invention include, but are not limited to, a dye, pigment, lake, effect pigment, and mixture thereof. Typical suitable colorants for eye-area cosmetics include any organic or inorganic pigment or colorant approved for use in eye-area cosmetics by CTFA and/or the FDA such as lakes, iron oxides, titanium dioxide, iron sulfides, ultramarines or other conventional pigments used in cosmetic formulations.

Examples of pigments include inorganic pigments such as chromium oxide greens, ultramarines, yellow iron oxide, brown iron oxide, red iron oxide, and titanium oxide; and organic pigments such as D&C Black No. 2, D&C Black No. 3, FD&C Red No. 40, D&C Green No. 5, FD&C Blue No. 1, and FD&C Yellow No. 5. Examples of lake dyes include various acid dyes which are laked with aluminum, calcium or barium.

The above pigments, dyes, and lakes are well known, commercially available materials, with their chemical structure being described, e.g., in 21 C. F. R. Part 74 (as revised April 1, 1988) and in the CTFA Cosmetic Ingredient Handbook, (1988), published by the Cosmetics, Toiletry and Fragrances Association, Inc.

Encapsulated colorant microparticles having average diameters of 0.1 to 10 microns are acceptable for use in the compositions of the present invention, for example 0.1 to 5 and especially 0.1 to 1 microns. The encapsulated colorant microparticles may comprise 1 to 60% by weight of at least one colorant, for example 5- 40% and especially 7 to 25% by weight.

Additionally, the microencapsulated colorants may provide a more vibrant color to products used around the eye area, including eyelashes. The primary colors are understood to Encapsulated colorant microparticles having average diameters of 0.1 to 10 microns are acceptable for use in the compositions of the present invention, for example 0.1 to 5 and especially 0.1 to 1 microns. The encapsulated colorant microparticles may comprise 1 to 60% by weight of at least one colorant, for example 5- 40% and especially 7 to 25% by weight.

Additionally, the microencapsulated colorants may provide a more vibrant color to products used around the eye area, including eyelashes. The primary colors are understood to mean red, yellow and blue. An additional feature of microparticles is the elimination of milling or grinding often encountered with non-encapsulated colorants. Said colorants

are preferably organic.

Colorants that are surface modified with a hydrophobic coating are acceptable for use in the compositions of the present invention, for example Triethoxycaprylsilane. Hydrophobically coating of colorants may increase their dispensability in the non-polar solvent and increase their resistance to being washed off during exposure to showering and facial cleansing.

The colorant for use herein is preferably selected from the following exemplary list of currently available colorants approved by the FDA for use around the eyes. Other colorants may also be used as they are developed and determined safe. Colorants for use in the present invention can be selected from the group consisting of annatto, caramel, carmine, ß-carotene, bismuth oxychloride, iron oxides, ferric ammonium ferrocyanide, ferric ferrocyanide, chromium hydroxide green, chromium oxide greens, guanine, mica, titanium dioxide, aluminum powder, bronze powder, copper powder, ultramarines, manganese violet, zinc oxide, D&C Black No. 2, D&C Black No. 3, FD&C Blue No. 1, D&C Green No. 5, FD&C Red No. 40, and FD&C Yellow No. 5.

Effect pigments useful in the present invention include, but are not limited to, pearls, nacres, and the effect pigments described in US2009/0220557 to Pfaff and US 6,875,264 to Zimmerman.

The mascara composition according to the invention comprises from 0.5 to 50% by weight, and preferably from 0.5 to 35% by weight based on the total weight of the composition, of a colorant, more preferably from 0.1% to 14%. Colorants having average diameters of 0.1 to 10 microns are acceptable for use in the compositions of the present invention, for example 0.1 to 5 and especially 0.1 to 1 microns. It is preferable that the diameter of the colorant is smaller than the thickness of the mascara composition dried-down film. The preferred diameter of the colorant is less than 1 micron. The small size of the colorants allows them to be fully encased in the dried film. In certain embodiments, if effect pigments are used, average diameters in excess of 30 microns are acceptable for use.

***Thickeners***

The mascara composition can be thickened or structured with colloidal particles. Thickeners for use in the present invention can be selected from the group consisting of particles such as disteardimonium hectorite, kaolin, silica, and magnesium carbonate; polymers; viscous hydrocarbons; and combinations thereof. The mascara composition is substantially free of wax.

Disteardimonium hectorite is the preferred thickener to build structure/viscosity. This enables proper spreading/deposition across lashes, and ensures adequate stability/suspension of colorant particles in dispersion over time. It is preferable that the diameter of the disteardimonium hectorite is smaller than the thickness of the mascara composition dried-down film. The preferred diameter of the disteardimonium hectorite is less than 10 microns. The mascara composition according to the present invention comprises from 1% to 25% disteardimonium hectorite, preferably from 2% to 20% disteardimonium hectorite, most preferably from 3% to 15% disteardimonium hectorite.

***Mascara Top Coat***

A second composition may be placed over the mascara compositions of the present invention or over any commercially available mascara. These second, overlying compositions are referred to herein as a top coat. The preferred topcoat composition is anhydrous and designed to mitigate the tackiness of the base layer or basecoat as well as extend the wear of the product.

An essential component of the top coat is a film forming polymer. One example of a film forming polymer suitable for use in the present invention is an organosiloxane resin. The film forming polymers can also be incorporated into the mascara composition described above. The resin may comprise combinations of $R_3SiO_{1/2}$ "M" units, $R_2SiO$ "D" units, $RSiO_{3/2}$ "T" units, $SiO_2$ "Q" units in ratios to each other that satisfy the relationship $R_nSiO_{(4-n)/2}$ where n is a value between 1.0 and 1.50 and R is a methyl group. Up to 5% of silanol or alkoxy functionality may also be present in the resin structure as a result of processing. The "M" to "Q" functional units is 0.6 to 2.0, more preferably 0.6 to 0.9, most preferably 0.7. Examples of organosiloxane resins commercially available are Wacker 803 and 804 available from Wacker Silicones Corporation of Adrian Michigan, and G.E. SR1000 from the General Electric Company.

Organosiloxane resins are used in the present invention at levels from 10% to 95%, preferably from 55% to 80%, and most preferably 60% to 70% of the total amount of top coat composition. In one embodiment of this invention the top coat comprises less than 3.0%, preferably less than 1.0%, even more preferably less than 0.1%, by weight, of colorants.

In a preferred embodiment, isododecane is used as a volatile solvent and trimethylsiloxysilicate (MQ Resin) and dimethicone are used as film-forming agents. These ingredients were selected because in combination they form flexible and non-tacky films.

*Mascara Remover*

As discussed above, high concentrations of PHR and tall oils substantially reduce the solubility of the mascara in soap and water. Prior mascara formulations kept the concentration of these two components low to insure the mascara could be washed off in soap and water. Accordingly, in one aspect of this invention a solvent-based mascara-remover composition is provided. The mascara remover comprises at least 40%, preferably at least 50%, and even more preferably at least 55% of a solvent selected from the group consisting of mineral oil, petroleum jelly, isododecane, silicones, other hydrocarbon solvents and mixtures thereof. The solvent can comprise up to 100% of the remover composition.

*Optional Ingredients*

The mascara, top coat, and mascara remover described above may contain other optional ingredients. Because there is some overlap between the mascara, top coat, and remover compositions described above and the optional ingredients which may be included in them, the optional ingredients are described together below.

*Actives*

The compositions of the present invention may comprise a safe and effective amount of a biological, chemical, nutraceutical, or pharmaceutical active, or a combination thereof. Biological actives may include prostaglandins, antimicrobials, antibacterials, biocides, preservatives, proteins, amino acids, peptides, hormones, growth factors, enzymes (e.g., glutathione sulphydryl oxidase, transglutaminase), therapeutics, oligonucleotides, genetic materials (e.g., DNA, RNA), and combinations thereof. Chemical actives may include dyes, surfactants, sensates, hair conditioners, hair dyes, hair growth agents, hair removers, hair growth inhibitors, hair styling gels, and combinations thereof. Nutraceutical actives may include proteins, preservatives, vitamins, food-additive materials, and combinations thereof. Pharmaceutical actives may include antibiotics, drugs, hair growth agents, hair removers, hair growth inhibitors, and combinations thereof.

*Oil Soluble or Oil Dispersible Additives*

The choice of oil-soluble or dispersible additive and the amount present according to the invention will depend on the intended use of the composition and the effectiveness of the compound. In mascara, top coat and remover compositions, the oil-soluble or dispersible additive chosen is acceptable for skin and eye contact, as is well known to the skilled formulator. Suitable oil-soluble or dispersible additives are incorporated at levels generally between 1 and 20% by weight based on the weight of the matrix bead (equivalent to 90 to 300 % on weight of the colorant). Preferably 5 to 15% by weight of the oil-soluble or dispersible additive is employed.

The oil-soluble or dispersible additive may include fatty alcohols such as GUERBET alcohols based on fatty alcohols having from 6 to 30, preferably from 10 to 20 carbon atoms including lauryl alcohol, cetyl alcohol, stearyl alcohol, cetearyl alcohol, oleyl alcohol, benzoates of $C_{12}$-$C_{15}$ alcohols, acetylated lanolin alcohol, etc. Especially suitable is stearyl alcohol.

The oil-soluble or dispersible additive may include fatty acids such as Linear fatty acids of $C_6$-$C_{24}$, branched $C_6$-$C_{13}$carboxylic acids, hydroxycarboxylic acids, caproic acid, caprylic acid, 2-ethylhexanoic acid, capric acid, lauric acid, isotridecanoic acid, myristic acid, palmitic acid, palmitoleic acid, stearic acid, isostearic acid, oleic acid, elaidic acid, petroselinic acid, linoleic acid, linolenic acid, elaeostearic acid, arachidic acid, gadoleic acid, behenic acid and erucic acid and technical-grade mixtures thereof (obtained, for example, in the pressure removal of natural fats and oils, in the reduction of aldehydes from Roelen's oxosynthesis or in the dimerization of unsaturated fatty acids).

Further components that can be used are dicarboxylic acids of $C_2$-$C_{12}$, such as adipic acid, succinic acid, and maleic acid. Aromatic carboxylic acids, saturated and/or unsaturated, especially benzoic acid, can be used.

Additional components that can be used as the oil soluble or dispersible additive include carboxylic acid salts: for example the salts of $C_8$-$C_{24}$, preferably $C_{14}$-$C_{20}$ saturated or unsaturated fatty acids, $C_8$-$C_{22}$ primary or secondary alkyl sulfonates, alkyl glycerol sulfonates, the sulfonated polycarboxylic acids described in published British Patent 1,082,179, paraffin sulfonates, N-acyl, N'-alkyl taurates, alkyl phosphates, isethionates, alkyl succinamates, alkyl sulphosuccinates, monoesters or diesters of sulfosuccinates, N-acyl sarcosinates, alkyl glycoside sulfates, polyethoxycarboxylates, the cation being an alkali metal (sodium, potassium, lithium), an unsubstituted or substituted ammonium residue (methyl, dimethyl, trimethyl, tetramethyl ammonium, dimethyl piperidinium, etc.) or a derivative of an alkanol amine (monoethanol amine, diethanol amine, triethanol amine, etc.); alkaline soaps of sodium, potassium and ammonium; metallic soaps of calcium or magnesium; organic basis soaps such as lauric, palmitic, stearic and oleic acid, etc., alkyl phosphates or phosphoric acid esters: acid phosphate, diethanolamine phosphate, potassium cetyl phosphate.

***Silicones and Siloxanes***

Silicones or siloxanes (organosubstituted polysiloxanes) may be used herein. This includes, but is not limited to, dimethylpolysiloxanes, methylphenylpolysiloxanes, cyclic silicones, and also amino-, fatty acid-, alcohol-, polyether-, epoxy-, fluorine-, glycoside- and/or alkyl-modified silicone compounds, which at room temperature may be in either liquid or resinous form; linear polysiloxanes: dimethicones such as Dow Corning® 200 fluid, Mirasil® DM (Rhodia), dimethiconol; cyclic silicone fluids: cyclopentasiloxanes, volatiles such as Dow Corning® 345 fluid, Silbione® grade, Abil® grade; phenyltrimethicones; Dow combing® 556 fluid. Also suitable are simethicones, which are mixtures of dimethicones having an average chain length of from 200 to 300 dimethylsiloxane units with hydrogenated silicates. A detailed survey by Todd et al. of suitable volatile silicones may be found in addition in Cosm. Toil. 91, 27 (1976). Especially suitable are ethoxylated propoxylated dimethicone (e.g. Dow Corning 5225C Formulation Aid) and aminopropyldimethicone (e.g. Tinocare SiAl from Ciba Specialty Chemicals).

Fluorinated or perfluorinated alcohols and acids may be used herein. This includes, but is not limited to, perfluordodecanoic acid, perfluordecanoic acid, perfluoro-tert-butyl alcohol, perfluoroadipic acid, 2-(perfluoroalkyl)ethanol (ZONAL® BA-L).

The oil-soluble or dispersible additive may be an anionic surfactant. Examples of such anionic surfactants include: alkyl ester sulfonates of the formula $R_{100}$--CH($SO_3$M)-COO$R_{200}$, where $R_{100}$ is a $C_8$-$C_{20}$, preferably $C_{10}$-$C_{16}$ alkyl radical, $R_{200}$ is a $C_1$-$C_{16}$, preferably $C_1$-$C_3$ alkyl radical, and M is an alkaline cation (sodium, potassium, lithium), substituted or non-substituted ammonium (methyl, dimethyl, trimethyl, tetramethyl ammonium, dimethyl piperidinium, etc.) or a derivative of an alkanol amine (monoethanol amine, diethanol amine, triethanol amine, etc.); alkyl sulfates of the formula $R_{300}OSO_3M$, where $R_{300}$ is a $C_5$-$C_{24}$, preferably $C_{10}$-$C_{18}$ alkyl or hydroxyalkyl radical, and M is a hydrogen atom or a cation as defined above, and their ethyleneoxy (EO) and/or propyleneoxy (PO) derivatives, having on average 0.5 to 30, preferably 0.5 to 10 EO and/or PO units; alkyl amide sulfates of the formula $R_{400}CONHR_{500}OSO_3M$, where $R_{400}$ is a $C_2$-$C_{22}$, preferably $C_6$-$C_{20}$ alkyl radical, $R_{500}$ is a $C_2$-$C_3$ alkyl radical, and M is a hydrogen atom or a cation as defined above, and their ethyleneoxy (EO) and/or propyleneoxy (PO) derivatives, having on average 0.5 to 60 EO and/or PO units.

The oil-soluble or dispersible additive may be a non-ionic surfactant. Nonionic surfactants that may be used include the primary and secondary alcohol ethoxylates, especially the $C_8$-$C_{20}$ aliphatic alcohols ethoxylated with an average of from 1 to 20 moles of ethylene oxide per mole of alcohol, and more especially the $C_{10}$-$C_{15}$ primary and secondary aliphatic alcohols ethoxylated with an average of from 1 to 10 moles of ethylene oxide per mole of alcohol. Non-ethoxylated nonionic surfactants include alkylpolyglycosides, glycerol monoethers, and polyhydroxyamides (glucamides).

Some particular examples of such nonionic surfactants include: polyalkoxylenated alkyl phenols (i.e. polyethyleneoxy, polypropyleneoxy, polybutyleneoxy), the alkyl substituent of which has from 6 to 12 C atoms and contains from 5 to 25 alkoxylenated units; examples are TRITON X-45, X-114, X-100 and X-102 marketed by Rohm & Haas Co., and IGEPAL NP2 to NP17 made by Rhodia; $C_8$-$C_{22}$ polyalkoxylenated aliphatic alcohols containing 1 to 25 alkoxylenated (ethyleneoxy, propyleneoxy) units; examples include TERGITOL 15-S-9, TERGITOL 24-L-6 NMW marketed by Dow, NEODOL 45-9, NEODOL 23-65, NEODOL 45-7, and NEODOL 45-4 marketed by Shell Chemical Co., KYRO EOB marketed by The Procter & Gamble Co., SYNPERONIC A3 to A9 made by ICI, RHODASURF IT, DB and B made by Rhodia; the products resulting from the condensation of ethylene oxide or propylene oxide with propylene glycol and/or ethylene glycol, with a molecular weight in the order of 2,000 to 10,000, such as the PLURONIC products marketed by BASF; the products resulting from the condensation of ethylene oxide and/or propylene oxide with ethylene diamine, such as the TETRONIC products marketed by BASF; $C_8$-$C_{18}$ ethoxyl and/or propoxyl fatty acids containing 5 to 25 ethyleneoxy and/or propyleneoxy units; $C_8$-$C_{20}$ fatty acid amides containing 5 to 30 ethyleneoxy units; ethoxylated amines containing 5 to 30 ethyleneoxy units; alkoxylated amidoamines containing 1 to 50, preferably 1 to 25 and in particular 2 to 20 alkyleneoxy (preferably ethyleneoxy) units; amine oxides such as the oxides of alkyl $C_{10}$-$C_{18}$ dimethylamines, the oxides of alkoxy $C_8$-$C_{22}$ ethyl dihydroxy ethylamines; alkoxylated terpene hydrocarbons such as ethoxylated and/or propoxylated $\alpha$- or $\beta$ pinenes, containing 1 to 30 ethyleneoxy and/or propyleneoxy units; alkylpolyglycosides obtainable by condensation (for example by acid catalysis) of glucose with primary fatty alcohols (e.g. those in U.S. Patents No. 3,598,865 and 4,565,647; and EP-A-132 043 and EP-A-132 046) having a $C_4$-$C_{20}$, preferably $C_8$-$C_{18}$ alkyl group and an average number of glucose units in the order of 0.5 to 3, preferably in the order of 1.1 to 1.8 per mole of alkylpolyglycoside (APG), particularly those having a $C_8$-$C_{14}$ alkyl group and on average 1.4 glucose units per mole, a $C_{12}$-$C_{14}$ alkyl group and on average 1.4 glucose units per mole, a $C_8$-$C_{14}$ alkyl group and on average 1.5 glucose units per mole or a $C_8$-$C_{10}$ alkyl group and on average 1.6 glucose units per mole, marketed under the names GLUCOPON 600 EC, GLUCOPON 600 CSUP, GLUCOPON 650 EC and GLUCOPON 225 CSUP respectively and made by Henkel.

Another class of suitable surfactants comprises certain mono-long chain-alkyl cationic surfactants. Cationic surfactants of this type include quaternary ammonium salts of the general formula $R_{10}R_{20}R_{30}R_{30}R_{40}N^+ X^-$ wherein the R groups are long or short hydrocarbon chains; typically alkyl, hydroxyalkyl or ethoxylated alkyl groups, and X is a

counter-ion (for example, compounds in which $R_{10}$ is a $C_8$-$C_{22}$ alkyl group, preferably a $C_8$-$C_{10}$ or $C_{12}$-$C_{14}$ alkyl group, $R_{20}$ is a methyl group, and $R_{30}$ and $R_{40}$, which may be the same or different, are methyl or hydroxyethyl groups); and cationic esters (for example, choline esters).

Also useful are ethoxylated carboxylic acids or polyethylene glycol esters (PEG-n acylates), linear fatty alcohols having from 8 to 22 carbon atoms, products from 2 to 30 mol of ethylene oxide and/or from 0 to 5 mol propylene oxide with fatty acids having from 12 to 22 carbon atoms and with alkylphenols having from 8 to 15 carbon atoms in the alkyl group, fatty alcohol polyglycol ethers such as Laureth-n, Ceteareth-n, Steareth-n and Oleth-n, fatty acid polyglycol ethers such as PEG-n Stearate, PEG-n Oleate and PEG-n Cocoate; polyethoxylated or acrylated lanolin; monoglycerides and polyol esters; $C_{12}$-$C_{22}$ fatty acid mono- and di-esters of addition products of from 1 to 30 mol of ethylene oxide with polyols; fatty acid and polyglycerol esters such as monostearate glycerol, diisostearoyl polyglyceryl-3-diisostearates, polyglyceryl-3-diisostearates, triglyceryl diisostearates, polyglyceryl-2-sesquiisostearates or polyglyceryl dimerates. Mixtures of compounds from a plurality of these substance classes are also suitable. Fatty acid polyglycol esters such as monostearate diethylene glycol, fatty acid and polyethylene glycol esters; fatty acid and saccharose esters such as sucro esters, glycerol and saccharose esters such as sucro glycerides; sorbitol and sorbitan: sorbitan mono- and di-esters of saturated and unsaturated fatty acids having from 6 to 22 carbon atoms and ethylene oxide addition products; polysorbate-n series, sorbitan esters such as sesquiisostearate, sorbitan, PEG-(6)-isostearate sorbitan, PEG-(10)-laurate sorbitan, PEG-17- dioleate sorbitan; glucose derivatives: $C_8$-$C_{22}$ alkyl-mono and oligo-glycosides and ethoxylated analogues with glucose being preferred as the sugar component; O/W emulsifiers such as Methyl Gluceth-20 sesquistearate, sorbitan stearate/sucrose cocoate, methyl glucose sesquistearate, cetearyl alcohol/cetearyl glucoside; also W/O emulsifiers such as methyl glucose dioleate/methyl glucose isostearate.

Oil-soluble or dispersible additives also include sulfates and sulfonated derivatives: e.g. dialkylsulfosuccinates (e.g. DOSS, dioctyl sulfosuccinate), alkyl lauryl sulfonate, linear sulfonated paraffins, sulfonated tetrapropylene sulfonate, sodium lauryl sulfates, ammonium and ethanolamine lauryl sulfates, lauryl ether sulfates, sodium laureth sulfates, acetyl isothionates, alkanolamide sulfates such as taurines, methyl taurines, and imidazole sulfates.

Oil-soluble or dispersible additives also include amine derivatives: amine salts, ethoxylated amines such as amine oxides, amines with chains containing a heterocycle such as alkyl imidazolines, pyridine derivatives, isoquinolines, cetyl pyridinium chloride, cetyl pyridinium bromide, quaternary anmionium compounds such as cetyltrimethylanmionium bromide, and stearylalkonium salts; amide derivatives: alkanolamides such as acylamide DEA, ethoxylated amides, such as PEG-n acylamide, oxydeamide;polysiloxane/polyalkyl/polyether copolymers and derivatives: dimethicone, copolyols, silicone polyethylene oxide copolymers and silicone glycol copolymers; propoxylated or POE-n ethers (Meroxapols), Polaxamers or poly(oxyethylene)$_m$-block-poly(oxypropylene)$_n$-block(oxyethylene) copolymers; zwitterionic surfactants that carry at least one quaternary ammonium group and at least one carboxylate and/or sulfonate group in the molecule, zwitterionic surfactants that are especially suitable include the so-called betaines, such as N-alkyl-N,N-dimethylammonium glycinates, for example cocoalkyldimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazolines each having from 8 to 18 carbon atoms in the alkyl or acyl group and also cocoacylaminoethylhydroxyethyl-carboxy-methylglycinate, N-alkylbetaines and N-alkylaminobetaines; alkylimidazolines, alkylopeptides and lipoaminoacids; self-emulsifying bases (see K.F. DePolo - A Short Textbook Of Cosmetology, Chapter 8, Table 8-7, p 250-251); non-ionic bases such as PEG-6 Beeswax (and) PEG-6 stearate (and) polyglyceryl-2-isostearate [Apifac], Glyceryl stearate (and) PEG-100 stearate, [Arlacel 165], PEG-5 Glyceryl stearate [Arlatone 983 S], sorbitan oleate (and) polyglyceryl-3-ricinoleate [Arlacel 1689], sorbitan stearate and sucrose cocoate [Arlatone 2121], glyceryl stearate and laureth-23 [Cerasynth 945], cetearyl alcohol and Ceteth-20 [Cetomacrogol Wax], cetearyl alcohol and Polysorbate 60 and PEG-150 and stearate-20 [Polawax GP 200, Polawax NF], cetearyl alcohol and cetearyl polyglucoside [Emulgade PL 1618], cetearyl alcohol and Ceteareth-20 [Emulgade 1000NI, Cosmowax], cetearyl alcohol and PEG-40 castor oil [Emulgade F Special], cetearyl alcohol and PEG-40 castor oil and sodium cetearyl sulfate [Emulgade F], stearyl alcohol and Steareth-7 and Steareth-10 [Emulgator E 2155], cetearyl Alcohol and Steareth-7 and Steareth-10 [Emulsifying wax U.S.N.F], glyceryl stearate and PEG-75 stearate [Gelot 64], propylene glycol ceteth-3 acetate [Hetester PCS], propylene glycol isoceth-3 acetate [Hetester PHA], cetearyl alcohol and Ceteth-12 and Oleth-12 [Lanbritol Wax N 21], PEG -6 stearate and PEG-32 stearate [Tefose 1500], PEG-6 stearate and Ceteth-20 and Steareth-20 [Tefose 2000], PEG-6 Stearate and ceteth-20 and Glyceryl Stearate and steareth-20 [Tefose 2561], glyceryl stearate and Ceteareth-20 [Teginacid H, C, X]; anionic alkaline bases such as PEG-2 stearate SE, glyceryl stearate SE [Monelgine, Cutina KD] and propylene glycol stearate [Tegin P]; anionic acid bases such as cetearyl alcohol and sodium cetearyl sulfate [Lanette N, Cutina LE, Crodacol GP], cetearyl alcohol and sodium lauryl sulfate [Lanette W], Trilaneth-4 phosphate and glycol stearate and PEG-2 stearate [Sedefos 75], glyceryl stearate and sodium lauryl sulfate [Teginacid Special]; and cationic acid bases such as cetearyl alcohol and cetrimonium bromide.

Other useful oil-soluble or dispersible additives comprise mild surfactants, super-fatting agents, consistency regulators, additional thickeners, polymers, stabilizers, biologically active ingredients, deodorizing active ingredients, anti-dandruff agents, film formers, swelling agents, UV light-protective factors, antioxidants, preservatives, insect repellents, solubilizers, colorants, bacteria-inhibiting agents, hair conditioning agents, vitamins, and the like.

***Packaging***

The mascara composition of the present invention may be packaged alone or in combination with a basecoat, topcoat, remover, eyeliner, eyebrow product, or combination thereof. In one embodiment, a mascara kit comprises a double-ended package, wherein the first end contains a mascara composition of the present invention and wherein the second end contains a mascara-remover composition of the present invention. In another embodiment, a mascara kit comprises a double-ended package, wherein the first end contains a mascara composition of the present invention and wherein the second end contains a topcoat composition.

A mascara, basecoat, topcoat, eyeliner, or eyebrow-product package (or complementary advertising) of the present invention may comprise indicia which suggests the long-wearing nature of the mascara composition. For instance, the indicia may comprise a certain number of hours the product may last, for example, 24 hours, 36 hours, or 48 hours. Or, the indicia may comprise a clock graphic. The indicia may be in a form selected from the group consisting of a label, a graphic, a three-dimensional shape, and combinations thereof. In one embodiment, the package comprises an hourglass indicia; the hourglass indicia may signal to the consumer that the product within is long-lasting. In a preferred embodiment, a mascara kit comprises a double-ended package, wherein the first end contains a mascara composition of the present invention and wherein the second end contains a mascara-remover composition of the present invention, and wherein the double-ended package comprises an indicia suggesting long-wear. Most preferably, the indicia comprises an hourglass.

***Uses***

In addition to the conventional mascara uses (e.g., coloring the lashes), the mascara composition or topcoat of the present invention may be used for additional functions. For example, the mascara described herein may be used as an eyeliner, an eyebrow pencil, an eyebrow styling gel, an eyebrow highlighter, a basecoat, a primer, an active-delivery system, etc. The mascara composition described may also be used to attach adornments to the face or specifically, around the eye area. For instance, the mascara composition of the present invention may be used to attach false lashes, glitter, rhinestones, feathers, jewelry, etc to the face or eye area. Furthermore, the mascara composition of the present invention may be modified to create semi-permanent face makeup, such as foundation, concealer, blush, lipstick, etc, or to create a body concealer *(e.g.,* to cover tattoos, blemishes, veins, or bruises on various parts of the body, such as the legs or back). One of ordinary skill in the art will recognize that not all of these forms require the presence of a colorant, e.g., a composition to attach adornments or a brow styling gel may be clear.

TEST METHODS

In the Example section below, mascara compositions of the present invention are tested against current mascara compositions (products that are or were recently offered for sale to the public). Two test methods are used: the Flexibility Test and the Rub Test.

***Flexibility Test Method***

Flexibility is measured by the latex stretch test. This test predicts the ability of the color film (that is the mascara composition) to resist flaking and peeling after application. The film failure by flaking, peeling and other methods is generally caused by movement of the skin, eyelids and eyelashes during normal activities. Failure can also occur by physical abrasion, such as rubbing the eyes or washing the face. The latex stretch test method is as follows:

Equipment:

1. Ansell Edmont Industrial technicians unlined gloves (12" length, 17 mil) USDA Accepted #390, Size 9;
2. A disposable lip brush such as those available from La Femme Cosmetics, Inc. of L.A.
3. Analytical balance (4 decimal places);
4. Ruler; and
5. An apparatus as described and illustrated in Figure 2 of US Patent No. 6,071,503.
Said apparatus can be constructed from Lucite sheet and rod stock wherein posts 2a are approximately 11 inches apart.
6. Butler Gum Technique Full Size Head Toothbrush, Soft Bristles

Procedure:

(1) Cut a 1 inch wide band from the wrist area of the glove, avoiding the ribbing and thumb.
(2) Mark off a 1 x 1inch block in the center of the band, avoiding the embossed number.
(3) Using a disposable lip brush, evenly apply 40mg +/-2mg over the 1 x 1 inch area of the band as marked in step (2).
(4) Allow the sample on the latex band from step (6) to sit at ambient room conditions for 10 minutes. Then place the sample in a 50°C oven for 1 hour. After an hour, remove the sample from the oven and allow the sample to sit at ambient room conditions for 10 minutes.
(5) Weigh and record the combined weight of the latex band A and the applied cosmetic film; hereinafter referred to as A.
(6) Stretch the band to a length of 11 inches 5 times, allowing the band to return to its approximate original shape after each stretch. On the $5^{th}$ stretch, slip the band over the posts (2a) of apparatus (1)
(7) Rub the film surface with 5 strokes using the Gum toothbrush.
(8) Carefully remove the latex band from the posts (2a) allowing it to returns to its approximate original shape.
(9) Record the weight of the latex band (with the remaining cosmetic); herein referred to as B.
(10) Calculate the weight loss of the cosmetic film using the following equation:

$$\text{Weight Loss (WL)} = A - B$$

Steps (1) through (10) are repeated three times for each cosmetic formula tested. The average of the three WL values is determined; herein referred to as Average Weight Loss; or AWL. A Low AWL value corresponds to flexible films having desirable adhesive and cohesive balance of the film. The AWL for compositions of the present invention is 0.003g and less, most preferably 0.

***Rub Test Method***

This test predicts the ability of a cosmetic film to resist color transfer to objects contacting the eyelash. Such objects may include hands, clothing, handkerchiefs or tissues, napkins, wash clothes, towels and the like.

Equipment:

10" X 5½" Leneta Form 2A Opacity Charts
0.006" draw down bar
Draw down board
Kimberly-Clark "WypAll" Paper towels
2½" diameter 2kg weight
1½" diameter arch punch
Scissors
50°C Oven
Double sided tape
Datacolor Microflash 200d

Procedure:

1. Drawdown Preparation:

a. Place a Leneta card on a drawdown board, Black/White side up. Label the top right of the card with sufficient sample & solvent identification. Take care to touch the card as little as possible since skin oils can affect the film thickness of product on the card.
b. Apply 1-2ml of product in a line across the top of the card, and use a 0.006" drawdown bar to draw a film down the entire length of the card.
c. Repeat steps 1a & 1b for all products to be tested.
d. Once all draw downs have been completed, place them in a 50°C oven for one hour. Take care not to mar

the film surfaces.

e. After an hour, remove the samples from the oven and allow them to equilibrate to ambient room temperature.

2. Sample Abrasion:

a. Use an arch punch to punch out 1½" diameter "abrasion substrate" disks from "WypAll" paper towels.

b. Completely and evenly, (do not overlap) apply strips of double sided tape to the bottom of a 2kg weight. Cut away excess tape that extends beyond the perimeter of the weight bottom. Place and adhere a single WypAll disk (1 ply thickness) to the sticky bottom of the weight. This will be used to abrade the film and see how much of it is removed.

c. Place the disk/weight in the center of the first 3" x 3" testing area. Twist the disk through two full revolutions in 1/8 revolution increments. Carefully remove the weight (up and away from the drawdown), and remove the abrading disk from the bottom of the weight. (Only change the double sided tape when a disk will no longer firmly adhere to the bottom of the weight.)

d. Perform 2a, 2b, & 2c for all subsequent Leneta card drawdowns.

3. Color Measurement

a. Turn on the Microflash by flipping the toggle switch located on the back panel.

b. Press the 'Menu' key on the front of the microflash until the word "Set-up" appears.

c. Click the button directly below the word "set-up" to select the set-up menu.

d. Use the arrow keys to pick the calibration program.

e. Set the 'hand-held head' to Specular Included. (Using the toggles on the head, confirm that "white" (vs black) is showing through port.)

f. The calibration program prompts you to place the white tile under the microflash head and press the trigger.

g. The prompt that instructs you to measure the Black Trap. Place the black trap under the head and squeeze the trigger.

h. The calibration is now complete.

i. Using the toggles on the back of the Microflash's hand-held head, set the Specular to "Excluded". (Confirm that "black" is showing through the port.)

j. Select 'Illuminant' from the Main Menu. Use the D65/10 setting.

k. Select Display from the main menu. Select CIE LCH Data and choose add.

l. To take measurements, press the large Menu Key on the front of the Microflash.

m. Place the Microflash over an unused "WypAll" disc and take a reading by squeezing the trigger. Then place the Microflash over a "WypAll" disc that has abraded a product film and take a reading by squeezing the trigger.

n. Record the Delta L value (DL)

o. Wipe / clean measuring surface in between samples.

Steps (1) through (3) are repeated three times for each cosmetic formula tested. The average of the three DL values is determined; herein referred to as Average Delta L Loss or Avg. DL. Low Avg. DL values correspond to films having greater abrasion resistance. The Avg. DL for compositions of the present invention are 4.00 and less, preferably 3.00 and less, most preferably 2.00 or less.

MASCARA COMPOSITION EXAMPLES

EXAMPLE 1

A mascara composition according to the present invention is made having the composition of Table 1 according to the method given directly below Table 1.

Table 1

| Mascara Composition | | |
|---|---|---|
| **Phase** | **Material** | **Weight %** |
| A | Tall Oil Glycerides | 8.000 |
| A | Pentaerythrityl Hydrogenated Rosinate | 12.00 |

(continued)

| Mascara Composition | | |
|---|---|---|
| **Phase** | **Material** | **Weight %** |
| A | Trihydroxystearin | 2.500 |
| A | Propylparaben | 0.100 |
| A | BHA | 0.100 |
| A | Phenoxyethanol 99% | 0.800 |
| A | Petroleum Distillates | 57.50 |
| B | Disteardimonium Hectorite | 5.000 |
| C | Propylene Carbonate | 2.500 |
| D | Black Iron Oxide | 5.500 |
| E | Encapsulated Blue Pigment | 6.000 |
| TOTAL | | 100.000 |

Phase A ingredients are melted and mixed together with low shear mixing. Phase B is gradually added to the Phase A and then dispersed with high shear mixing. Phase C is then added and mixed in with high shear mixing. Phase D is then added and dispersed with high shear mixing. The batch is cooled to ambient conditions and then Phase E is added and mixed in.

EXAMPLE 2

Additional mascara compositions according to the present invention, Nos.1-4, are prepared and have the compositions given below in Table 2A.

Table 2A

| Inventive Mascara Compositions | | | | | |
|---|---|---|---|---|---|
| ***Raw Material Description*** | **Supplier/Trade Name** | **1 % W** | **2 %W** | **3 %W** | **4 %W** |
| Isododecane | Presperse Permthyl 99A | 46.00% | 46.00% | 54.00% | 56.00% |
| Pentaerythrityl Hydrogenated Rosinate | Eastman Foral 105-E | 12.50% | 12.50% | 8.50% | 7.50% |
| Tall Oil | Arizona Chemical Sylvagum RE 85K | 12.50% | 12.50% | 8.50% | 7.50% |
| Disteardimonium Hectorite | Elementis Bentone 38V CG | 14.00% | 14.00% | 14.00% | 14.00% |
| Propylene Carbonate | Huntsman Jeffsol | 4.50% | 4.50% | 4.50% | 4.50% |
| 1,2 Hexanediol, Caprylyl Glycol | Symrise Symdiol 68 | 1.00% | 1.00% | 1.00% | 1.00% |
| Silicone Treated Black Iron Oxide (Jet Milled) | Sensient Unipure Black LC 989 EM AS | 0 | 9.50% | 9.50% | 9.50% |
| Untreated Black Iron Oxide (Jet Milled) | Sensient Unipure Black LC 989 EM | 9.50% | 0 | 0 | 0 |
| | TOTAL | 100.00% | 100.00% | 100.00% | 100.00% |

Numerous current market mascaras, products recently available for sale to the general public, are purchased and tested. These products all advertise better or longer wear characteristics than standard mascara products. Regardless, the comparative mascara products that are tested are given in Table 2B below.

Table 2B

| Comparative Mascara Products | | |
|---|---|---|
| **Manufacturer** | **Mascara Name** | **Comparative Product No.** |
| Cover Girl® | Marathon® Water Proof | 5 |
| Cover Girl® | Lash Exact® | 6 |
| Avon®* | Perfect Wear® (16 Hour) | 7 |
| Sephora® | Tarte 4 day Stay Lash Stain® | 8 |
| Maybelline® | Great Lash® Water Proof | 9 |
| *This product is no longer available for sale to consumers; all other comparative products are available to consumers. | | |

The two test methods are described above and the products tested and the results of the tests are given below in Table 2C.

Table 2C

| Test Results | | |
|---|---|---|
| **Inventive Composition No.** | **Flexibility Test** | **Rub Test** |
| **1** | 0.0 mg | 2.13 ΔL |
| **2** | 0.0 mg | 0.78 ΔL |
| **3** | Not Tested | 1.70 ΔL |
| **4** | Not Tested | 0.29 ΔL |
| **Inventive Average** | **0.0 mg** | **1.23 ΔL** |
| **Comparative Product No.** | | |
| **5** | 2.0 mg | 4.64 ΔL |
| **6** | 4.0 mg | 4.76 ΔL |
| **7** | 12.0mg | 12.10 ΔL |
| **8** | 15.0mg | 11.66 ΔL |
| **9** | 6.0 mg | 10.85 ΔL |
| **Comparative Average** | **7.8 mg** | **8.80 ΔL** |

As is evident, the mascara compositions of this invention (Nos. 1-4) performed better individually and better on average than did any of the current market products (Nos. 5-9).

EXAMPLE 3

Another mascara composition is made according to the method below, having the composition according to Table 3.

Table 3

| Inventive Mascara Composition | | | | |
|---|---|---|---|---|
| **Phase** | **Material** | **10 Weight %** | **11 Weight %** | **12 Weight %** |
| A | Tall Oil Glycerides | 12.000 | 18.000 | 22.000 |

(continued)

| Phase | Material | 10 Weight % | 11 Weight % | 12 Weight % |
|---|---|---|---|---|
| A | Pentaerythrityl Hydrogenated Rosinate | 13.000 | 0.000 | 10.000 |
| A | Glyceryl Monostearate | 5.750 | 6.750 | 4.750 |
| A | Black Iron Oxide | 7.750 | 8.750 | 6.750 |
| A | Disteardimonium Hectorite | 2.750 | 2.750 | 2.750 |
| A | Stearic Acid | 2.750 | 2.750 | 2.250 |
| A | Triethanolamine | 1.750 | 1.750 | 1.250 |
| A | Polyvinyl Alcohol | 2.000 | 2.000 | 2.000 |
| A | Propylene Carbonate | 0.750 | 0.750 | 0.250 |
| A | Lecithin | 1.750 | 1.750 | 1.250 |
| A | Oleic Acid 80% | 1.500 | 1.500 | 1.500 |
| B | Acrylates Copolymer | 5.170 | 5.170 | 2.170 |
| B | Isododecane | 15.200 | 18.200 | 10.200 |
| B | Simethicone Emulsion 30% | 0.200 | 0.200 | 0.200 |
| C | Xanthan Gum | 0.600 | 0.600 | 0.600 |
| C | Propylene Glycol | 3.500 | 3.500 | 3.500 |
| D | Ammonium Acrylates Copolymer | 17.800 | 17.800 | 12.800 |
| E | Ethyl Alcohol SD 40-B | 1.000 | 1.000 | 1.000 |
| E | Benzyl Alcohol | 0.650 | 0.650 | 0.650 |
| E | Panthenol | 0.280 | 0.280 | 0.280 |
| E | Phenoxyethanol | 0.250 | 0.250 | 0.250 |
| E | Methylparaben | 0.200 | 0.200 | 0.200 |
| E | Ethylparaben | 0.200 | 0.200 | 0.200 |
| E | Propylparaben | 0.100 | 0.100 | 0.100 |
| E | Trisodium EDTA | 0.100 | 0.100 | 0.100 |
| F | Encapsulated Blue Pigment | 3.00 | 3.00 | 3.00 |
| | TOTAL | 100.000 | 100.000 | 100.000 |

Phase A is heated to allow the pigment to be dispersed with a Cowles Blade mixer. Phase B materials are stirred together at ambient conditions, and Phase C materials are stirred together at ambient conditions and then it is added to Phase B (to gel Phase B), and the mixture is stirred and then heated to 85°C. Phase A and Phases B/C are mixed together to create an oil (wax) in water emulsion. The mixture is stirred for 15 minutes and then is cooled gradually to room temperature. During the cool down, Phases D and E are added to the mixture and stirred in below 60°C. Phase F is added to and mixed with the mascara once the mascara has cooled down to 25-50°C.

EXAMPLE 4

Table 4 lists the composition for an exemplary top coat for use over the mascara compositions of the present invention.

Table 4

| Top Coat Composition | | | |
|---|---|---|---|
| **Raw Material Description** | **Supplier/Trade Name** | **Function** | **% Wt/Wt** |
| Isododecane | Presperse Permthyl 99A | Volatile Solvent | 49.995% |
| Trimethylsiloxysilicate | Momentive MQ Resin | Film Former | 38.025% |
| Dimethicone | Momentive SE-30 Gum | Film Former | 10.980% |
| 1,2 Hexanediol, Caprylyl Glycol | Symrise Symdiol 68 | Preservative | 1.000% |
| | | TOTAL | 100.000% |

EXAMPLE 5

Table 5 lists exemplary mascara compositions for use as eyeliners and concealer.

Table 5

| Raw Material Description | | Liquid Eyeliner Weight % | Concealer Weight % |
|---|---|---|---|
| Isododecane | | 51 | 56.510 |
| 1,2 Hexanediol and Caprylyl Glycol | | 1 | 1 |
| Tall Oil Glycerides | | 15 | 12.5 |
| Pentaerythrityl Hydrogenated Rosinate | | 15 | 12.5 |
| Black Iron Oxide & Silica (Jet Milled) | | 10 | 0.270 |
| Red Iron Oxide & Silica (Jet Milled) | | 0 | 0.580 |
| Yellow Iron Oxide & Silica (Jet Milled) | | 0 | 0.940 |
| Titanium Oxide | | 0 | 7.700 |
| Disteardimonium Hectorite | | 8 | 8 |
| Polyethylene wax | | 0 | 0 |
| TOTAL | | 100 | 100 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

**Claims**

1. A method of coating eyelids or eyelashes comprising the step of applying to eyelashes, eyelids, or both, a mascara composition comprising:

   a. from 15% to 50%, by weight, of a component selected from the group consisting of tall oil glycerides, pentaerythrityl rosinate, glyceryl rosinate, and the hydrogenated versions and mixtures thereof;
   b. from 0.5% to 50%, by weight, of a colorant; and
   c. the composition is substantially free of wax.

2. The method according to claim 1, wherein the composition scores less than 1.0 in a Flexibility Test, as disclosed in the present description.

3. The method according to claim 1 or 2, wherein the composition scores less than 3.0, most preferably less than 2.5, in a Rub Test, as disclosed in the present description.

4. The method according to any of the preceding claims, wherein the composition remains on the eyelashes or eyelids for more than 24 hours, more preferably more than 36 hours, most preferably more than 48 hours.

5. The method according to any of the preceding claims, wherein the mascara composition comprises from 17% to 30%, by weight, of a component selected from the group consisting of tall oil glyceride, pentaerythrityl hydrogenated rosinate, and mixtures thereof.

6. The method according to any of the preceding claims, further comprising the step of applying to the eyelash or eyelid a second layer comprising a clear polymer selected from the group consisting of organosiloxane resins and mixtures thereof.

7. The method according to claim 6, wherein the second layer comprises no colorant.

8. The method according to any of the preceding claims, wherein the mascara composition comprises from 15% to 50%, by weight, of one or more tall oils.

9. The method according to any of the preceding claims, wherein the mascara composition comprises from 1% to 20%, by weight, of disteardimonium hectorite.

10. The method according to any of the preceding claims, wherein the mascara composition is contained by a mascara package, wherein the mascara package comprises indicia suggesting long wear.

11. The method according to claim 10, wherein the indicia comprises an hourglass.

12. A mascara kit comprising a double-ended package, wherein the first end contains a mascara composition according to any of the preceding claims and the second end contains a mascara-remover composition.

13. The mascara kit of claim 12, wherein the package comprises an hourglass indicia suggesting long wear.

14. The method according to any of the preceding claims, wherein the mascara composition comprises a volatile carrier, wherein the ratio of film former to volatile carrier is controlled such that the dried mascara composition film consists of from 30% to 70% film former by weight, more preferably from 15% to 80%, and most preferably from 20% to 70%.

**Patentansprüche**

1. Verfahren zum Bestreichen von Augenlidern oder Augenwimpern, umfassend den Schritt des Aufbringens auf die Augenwimpern, die Augenlider oder auf beides einer Wimperntuschezusammensetzung, die folgendes umfasst:

   a. von 15 Gew.-% bis 50 Gew.-% eines Bestandteils, der ausgewählt ist aus der Gruppe bestehend aus Tallölglyceriden, Pentaerythritylrosinat, Glycerylrosinat und Mischungen davon;
   b. von 0,5 Gew.-% bis 50 Gew.-% eines Farbmittels; und

c. die Zusammensetzung ist im Wesentlichen frei von Wachs.

2. Verfahren nach Anspruch 1, wobei die Zusammensetzung in einem Flexibilitätstest, wie in der vorliegenden Beschreibung offenbart, bei unter 1,0 liegt.

3. Verfahren nach Anspruch 1 oder 2, wobei die Zusammensetzung in einem Reibtest, wie in der vorliegenden Beschreibung offenbart, unter 3,0, am meisten bevorzugt unter 2,5 liegt.

4. Verfahren nach einem der vorstehenden Ansprüche, wobei die Zusammensetzung für mehr als 24 Stunden, mehr bevorzugt mehr als 36 Stunden, am meisten bevorzugt mehr als 48 Stunden auf den Augenwimpern oder Augenlidern verbleibt.

5. Verfahren nach einem der vorstehenden Ansprüche, wobei die Wimperntuschezusammensetzung von 17 Gew.-% bis 30 Gew.-% einen Bestandteil umfasst, der ausgewählt ist aus der Gruppe bestehend aus Tallölglycerid, hydriertem Pentaerythritylrosinat und Mischungen davon.

6. Verfahren nach einem der vorstehenden Ansprüche, ferner umfassend den Schritt des Aufbringens auf die Augenwimpern oder Augenlider einer zweiten Schicht, die ein durchsichtiges Polymer umfasst, das ausgewählt ist aus der Gruppe bestehend aus Organosiloxanharzen und Mischungen davon.

7. Verfahren nach Anspruch 6, wobei die zweite Schicht kein Farbmittel umfasst.

8. Verfahren nach einem der vorstehenden Ansprüche, wobei die Wimperntuschezusammensetzung von 15 Gew.-% bis 50 Gew.-% ein oder mehrere Tallöle umfasst.

9. Verfahren nach einem der vorstehenden Ansprüche, wobei die Wimperntuschezusammensetzung von 1 Gew.-% bis 20 Gew.-% Disteardimoniumhectorit umfasst.

10. Verfahren nach einem der vorstehenden Ansprüche, wobei die Wimperntuschezusammensetzung in einer Wimperntuscheverpackung enthalten ist, wobei die Wimperntuscheverpackung einen Hinweis auf lange Haltbarkeit nach dem Auftragen umfasst.

11. Verfahren nach Anspruch 10, wobei der Hinweis eine Sanduhr umfasst.

12. Wimperntusche-Kit, eine Packung mit zwei Seiten umfassend, wobei die erste Seite eine Wimperntuschezusammensetzung nach einem der vorstehenden Ansprüche enthält und die zweite Seite eine Wimperntuscheentfernerzusammensetzung umfasst.

13. Wimperntusche-Kit nach Anspruch 12, wobei die Verpackung einen sanduhrförmigen Hinweis auf lange Haltbarkeit nach dem Auftragen umfasst.

14. Verfahren nach einem der vorstehenden Ansprüche, wobei die Wimperntuschezusammensetzung einen flüchtigen Träger umfasst, wobei das Verhältnis von Filmbildner zu flüchtigem Träger so gesteuert wird, dass der getrocknete Film aus der Wimperntuschezusammensetzung, bezogen auf das Gewicht, von 30 % bis 70 %, mehr bevorzugt von 15 % bis 80 % und am meisten bevorzugt von 20 % bis 70 % Filmbildner umfasst.

## Revendications

1. Procédé de maquillage des paupières ou des cils comprenant l'étape consistant à appliquer sur les cils, les paupières ou les deux, une composition de mascara comprenant :

   a. de 15 % à 50 % en poids d'un composant choisi dans le groupe constitué de glycérides de tallöl, de résinate de pentaérythrityle, de résinate de glycéryle, ainsi que des versions hydrogénées et mélanges de ces éléments ;
   b. de 0,5 % à 50 % en poids d'un colorant ; et
   c. la composition est essentiellement dépourvue de cire.

2. Procédé selon la revendication 1, dans lequel la composition obtient une note inférieure à 1,0 dans un essai de

flexibilité, comme décrit dans la présente description.

**3.** Procédé selon la revendication 1 ou 2, dans lequel la composition obtient une note inférieure à 3,0, de préférence inférieure à 2,5, dans un essai de frottement, comme décrit dans la présente description.

**4.** Procédé selon l'une quelconque des revendications précédentes, dans lequel la composition tient sur les cils ou les paupières plus de 24 heures, de préférence plus de 36 heures, idéalement plus de 48 heures.

**5.** Procédé selon l'une quelconque des revendications précédentes, dans lequel la composition de mascara comprend de 17 % à 30 % en poids d'un composant choisi dans le groupe constitué de glycéride de tallöl, de résinate hydrogéné de pentaérythrityle, ainsi que leurs mélanges.

**6.** Procédé selon l'une quelconque des revendications précédentes, comprenant en outre l'étape consistant à appliquer sur les paupières ou les cils une deuxième couche comprenant un polymère transparent choisi dans le groupe constitué par les résines d'organosiloxane et leurs mélanges.

**7.** Procédé selon la revendication 6, dans lequel la deuxième couche ne comprend pas de colorant.

**8.** Procédé selon l'une quelconque des revendications précédentes, dans lequel la composition de mascara comprend de 15 % à 50 % en poids d'une ou plusieurs huiles tallöl.

**9.** Procédé selon l'une quelconque des revendications précédentes, dans lequel la composition de mascara comprend de 1 % à 20 % en poids de disteardimonium hectorite.

**10.** Procédé selon l'une quelconque des revendications précédentes, dans lequel la composition de mascara est contenue dans un conditionnement de mascara, le conditionnement de mascara comprenant une marque distinctive suggérant une tenue de longue durée.

**11.** Procédé selon la revendication 10, dans lequel la marque distinctive comprend un sablier.

**12.** Kit de mascara comprenant un conditionnement à double extrémité, dans lequel la première extrémité contient une composition de mascara selon l'une quelconque des revendications précédentes et la deuxième extrémité contient une composition de démaquillage pour le mascara.

**13.** Kit de mascara selon la revendication 12, dans lequel le conditionnement comprend une marque distinctive constituée d'un sablier suggérant une tenue de longue durée.

**14.** Procédé selon l'une quelconque des revendications précédentes, dans lequel la composition de mascara comprend un véhicule volatil, le rapport entre la substance filmogène et le véhicule volatil étant contrôlé de telle sorte que le film de composition de mascara séché comprenne de 30 % à 70 % en poids, de préférence de 15 % à 80 %, et idéalement de 20 % à 70 % en poids de substance filmogène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20090220557 A, Pfaff
- US 6875264 B, Zimmerman
- BG 1082179
- US 3598865 A
- US 4565647 A
- EP 132043 A
- EP 132046 A
- US 6071503 A


### Non-patent literature cited in the description


- **K.F. DEPOLO.** A Short Textbook Of Cosmetology. 250-251